# EUROPEAN PATENT APPLICATION

(11) **EP 3 437 709 A1**
(43) Date of publication of application: **06.02.2019**
(21) Application number: 17774353.1
(22) Date of filing: 15.03.2017
(51) Int. Cl.: B01D 39/14, C07C 17/42, C07C 19/08

(54) **FILTER, METHOD FOR PRODUCING SAME, DRY ETCHING APPARATUS AND DRY ETCHING METHOD**

(30) Priority: 30.03.2016 JP 2016068869
(71) Applicant: Zeon Corporation, Tokyo 100-8246 (JP)
(72) Inventor: HYAKUTAKE Munehiro, Tokyo 100-8246 (JP); HIRANO Takaaki, Tokyo 100-8246 (JP)
(74) Representative: Adam, Holger
(86) International application number: PCT/JP2017/010525
(87) International publication number: WO 2017/169809

(57) **Abstract**

The present disclosure provides a filter that includes a portion that comes into contact with a filtering object and is at least partially made of metal or metal compound, wherein the filter is capable of suppressing decomposition of fluorinated saturated hydrocarbon when filtering a composition containing high concentration of fluorinated saturated hydrocarbon. The filter according to the present disclosure includes: an area made of metal or metal compound for coming into contact with the filtering object; and amine compound absorbed to a surface of the area. A method for producing a filter according to the present disclosure includes a step of bringing amine compound into contact with the surface of the area made of metal or metal compound for coming into contact with the filtering object.

## Description

### TECHNICAL FIELD

The present disclosure relates to a filter, a method for producing the filter, and an apparatus and a method for dry etching using the filter.

### BACKGROUND

Conventionally, a composition with high concentration (e.g. 99% by volume or more) of fluorinated saturated hydrocarbon such as 2-fluorobutane is used for an etching gas, a CVD gas, a raw material for producing an intermediate of a fluorine-containing pharmaceutical, a solvent, etc. (e.g., see PTL 1).

### CITATION LIST

### Patent Literature

PTL 1: WO2014/136877

### SUMMARY

### (Technical Problem)

Here, compositions with high concentration of fluorinated saturated hydrocarbon may be used for various applications after being filtered by a filter having a portion that comes into contact with a filtering object to be filtered and is at least partially made of metal or metal compound.

However, when a composite with high concentration, e.g., 99% by volume or more of fluorinated saturated hydrocarbon is filtered by a filter having a portion that comes into contact with a filtering object to be filtered and is at least partially made of metal or metal compound, the fluorinated saturated hydrocarbon may decompose and reduce its concentration.

Therefore, there is a demand for a filter, having a portion that comes into contact with a filtering object to be filtered and is at least partially made of metal or metal compound, to be able to suppress decomposition of fluorinated saturated hydrocarbon when filtering a composition with high concentration of fluorinated saturated hydrocarbon. Such a filter capable of suppressing decomposition of fluorinated saturated hydrocarbon is particularly required for a dry etching apparatus and a dry etching method which use a high-purity dry etching gas obtained by filtering a composition with high concentration of fluorinated saturated hydrocarbon.

As such, the present disclosure aims to provide a filter having a portion that comes into contact with a filtering object to be filtered and is at least partially made of metal or metal compound, wherein the filter is capable of suppressing decomposition of fluorinated saturated hydrocarbon when filtering a composition with high concentration of fluorinated saturated hydrocarbon.

The present disclosure also aims to provide a dry etching apparatus having such a filter and a dry etching method that uses the filter.

### (Solution to Problem)

The present inventors diligently studied in order to achieve the above object and completed the present disclosure by finding that using a filter having a portion that is made of metal or metal compound for coming into contact with a filtering object to be filtered and has amine compound adsorbed thereto may suppress decomposition of fluorinated saturated hydrocarbon when filtering a composition with high concentration of the fluorinated saturated hydrocarbon.

That is, in order to advantageously solve the above problem, a filter of the present disclosure includes: a portion made of metal or metal compound for coming into contact with a filtering object; and amine compound absorbed to a surface of the portion. When the portion made of metal or metal compound for coming into contact with the filtering object have a surface to which amine compound is absorbed as described above, the filter may suppress decomposition of fluorinated saturated hydrocarbon when filtering a composition with high concentration of fluorinated saturated hydrocarbon.

Here, according to the filter of the present disclosure, it is preferable that the amine compound is chemically adsorbed to the surface. The chemical absorption of the amine compound suppresses desorption of the amine compound from the surface of the portion made of metal or metal compound, thus preventing the amine compound from mixing into a filtrate obtained by filtering a filtering object.

According to the filter of the present disclosure, also, a desorption amount of the amine compound in a nitrogen gas atmosphere at a temperature of 20°C and a pressure (a gauge pressure) of 0.1 MPa is preferably 10 ppm by volume or less. When the desorption amount of the amine compound is 10 ppm by volume or less, desorption of the amine compound from the surface of the portion made of metal or metal compound is reduced, and the amine compound mixed into the filtrate obtained by filtering the filtering object may be reduced.

Note that the desorption amount of the amine compound can be measured by employing gas chromatography.

According to the filter of the present disclosure, a mesh size of a filter element is preferably at least 1,250. When the mesh size of the filter element is 1250 mesh or more, impurities such as particles contained in the filtering object may be satisfactorily removed.

According to the filter of the present disclosure, the amine compound is preferably composed of amine with 3 to 5 carbon atoms. The amine with 3 to 5 carbon atoms may be readily handled and is highly effective in suppressing decomposition of fluorinated saturated hydrocarbon.

Further, in order to advantageously solve the above problem, a method for producing a filter according to the present disclosure is a method for producing a filter having a portion that comes into contact with a filtering object to be filtered and is at least partially made of metal or metal compound, and includes a step (A) of bringing anime compound into contact with a surface of an area made of metal or metal compound. By bringing amine compound into contact with the surface of the area made of metal or metal compound as described above such that the amine compound is absorbed to the surface of the area, a filter capable of suppressing decomposition of fluorinated saturated hydrocarbon when filtering a composition with high concentration of fluorinated saturated hydrocarbon may be readily yielded.

Here, the method for producing the filter according to the present disclosure preferably further includes, after the step (A), a step (B) of removing the amine compound physically adsorbed to the surface. The removal of the amine compound physically absorbed to the surface suppresses desorption of the amine compound from the surface of the area made of metal or metal compound during filtration, and reduces amine compound mixed into the filtrate obtained by filtering the filtering object.

Preferably, the method for producing the filter according to the present disclosure further includes a step (B) of performing an operation for exposing the surface to a reduced pressure atmosphere. When the surface with which the amine compound is bought into contact is exposed to a reduced pressure atmosphere, the amine compound physically absorbed to the surface can be easily removed.

Preferably, the method for producing the filter according to the present disclosure includes a step (B) of alternately repeating the operation for exposing the surface to a reduced pressure atmosphere and an operation for bringing an inert gas into contact with the surface. When the operation for exposing the surface to a reduced pressure atmosphere and the operation for bringing the inert gas into contact with the surface are alternately repeated, the amine compound physically absorbed to the surface may be efficiently removed.

Further, in the method for producing the filter according to the present disclosure, a mesh size of a filter element of the filter is preferably at least 1,250 mesh. When the mesh size of the filter element is 1,250 mesh or more, impurities such as particles contained in the filtering object may be satisfactorily removed.

In the method for producing the filter according to the present disclosure, the amine compound is preferably composed of amine with 3 to 5 carbon atoms. Amine with 3 to 5 carbon atoms may be readily handled and is highly effective in suppressing decomposition of fluorinated saturated hydrocarbon.

Also, in order to advantageously solve the above problem, a dry etching apparatus according to the present disclosure is a dry etching apparatus for dry etching by using a dry etching gas filtered by any of the above filters. When a dry etching gas obtained by filtering a composition with high concentration of fluorinated saturated hydrocarbon using any of the above filters is used, decomposition of fluorinated saturated hydrocarbon is suppressed.

Further, in order to advantageously solve the above problem, a dry etching method according to the present disclosure performs dry etching by using a dry etching gas filtered by the filter described above. When the filter described above is used, decomposition of fluorinated saturated hydrocarbon is suppressed when a dry etching gas obtained by filtering a composition with high concentration of fluorinated saturated hydrocarbon is used.

### (Advantageous Effect)

The present disclosure may provide a filter having a portion that comes into contact with a filtering object and is at least partially made of metal or metal compound, wherein the filter is capable of suppressing decomposition of fluorinated saturated hydrocarbon when filtering a composition with high concentration of the fluorinated saturated hydrocarbon.

The present disclosure may also provide a dry etching apparatus having the filter capable of suppressing decomposition of fluorinated saturated hydrocarbon, and a dry etching method that uses the filter capable of suppressing decomposition of fluorinated saturated hydrocarbon.

### DETAILED DESCRIPTION

Hereinafter, an embodiment of the present disclosure will be described in detail.

Here, a filter of the present disclosure may be produced by any appropriate method including a method for producing a filter according to the present disclosure. A filter of the present disclosure and a filter produced by the method for producing the filter according to the present disclosure may be favorably used to filter a dry etching gas by, and not limited to, a dry etching apparatus and a dry etching method according to the present disclosure.

### Filter

The filter according to the present disclosure is used for, and not limited to, removal of fine particles contained in a filtering object to be filtered. The filter according to the present disclosure includes a portion made of metal or metal compound for coming into contact with the filtering object, and amine compound absorbed to a surface of the portion made of metal or metal compound.

The filter of the present disclosure includes amine compound absorbed to the surface of the portion made of metal or metal compound. Thus, when a composition containing high concentration, i.e., at least 99% by volume, preferably at least 99.50% by volume, more preferably at least 99.80% by volume, particularly preferably 99.9% by volume of fluorinated saturated hydrocarbon is filtered, decomposition of the fluorinated saturated hydrocarbon may be suppressed.

Here, it is believed that decomposition of fluorinated saturated hydrocarbon is suppressed for the following reason. That is, decomposition of fluorinated saturated hydrocarbon seems to occur when a Lewis acid component of metal atoms and the like on the surface of the portion made of metal or metal compound acts as a catalyst. However, when an amine compound absorbed to the surface is subjected to Lewis acid poisoning, a decomposition reaction (e.g., an HF elimination reaction) of fluorinated saturated hydrocarbon by the Lewis acid as a catalyst is suppressed, and thus the decomposition of fluorinated saturated hydrocarbon is suppressed.

### [Configuration]

Here, the filter according to the present disclosure may have any configuration as long as having a portion that comes into contact with a filtering object and is made of metal or metal compound. For example, the filter according to the present disclosure includes a filter body having a filtering object path to pass the filtering object therethrough, and a filter element for trapping fine particles and the like contained in a filtering object flowing in the filtering object path within the filter body. Each of the filter element and the filter body includes a portion that comes into contact with a filtering object to be filtered and is at least partially made of metal or metal compound. In particular, the filter of the present disclosure may be, and not limited to, a filter in which a filter element made of resin is provided within a filtering object path of a filter body made of metal or metal compound, a filter in which a filter element made of metal or metal compound is provided within a filtering object path of a filter body made of metal or metal compound, or a filter in which a filter element made of metal or metal compound is provided within a filtering object path of a filter body made of resin.

The metal mentioned above may be any appropriate metal including stainless steel or nickel.

The metal compound mentioned above may be any appropriate metal compound including aluminum oxide (alumina).

Further, the resin mentioned above may be any appropriate resin including polyethylene terephthalate (PTFE).

The filter element mentioned above may be fixedly or detachably provided within the filtering object path of the filter body.

Also, a filter element with any mesh size that demonstrates a desired filtration performance may be used. From the viewpoint of satisfactory removal of impurities such as particles contained in a filtering object, the mesh size of the filter element is preferably at least 1,250 mesh, more preferably at least 4,500 mesh.

### [Amine Compound]

The amine compound absorbed to the surface of the portion made of metal or metal compound may be any amine compound with 10 or less carbon atoms (as a total number of carbon atoms) expressed by any one of the following chemical formulas (I) to (III): 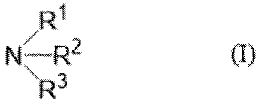 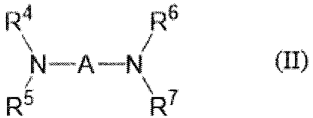 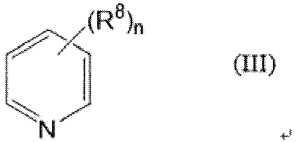 (wherein R¹ to R⁷ respectively represent a hydrogen atom, an alkyl group with 1 to 10 carbon atoms, a cycloalkyl group with 3 to 10 carbon atoms, or an aryl group with 6 to 10 carbon atoms; and R⁸ represents an alkyl group with 1 to 5 carbon atoms or a cycloalkyl group with 3 to 5 carbon atoms. Two groups selected from R¹ to R³ may be bonded to each other and form a ring. Also, R⁴ and R⁵ may be bonded to each other and form a ring, and R⁶ and R⁷ may be bonded to each other and form a ring. A represents a divalent group with 2 to 10 carbon atoms. N represents an integer of 0 to 5. When N is 2 or more, R⁸ may be identical to or different from each other.)

When the number of carbon atoms of the amine compound is 10 or less, the amine compound has a sufficiently low melting point and is in a liquid state or a gaseous state for enabling easy handling thereof. Thus, adsorption efficiency to the portion made of metal or metal compound may be improved.

The number of carbon atoms of the alkyl group represented by R¹ to R⁷ is 1 to 10, preferably 1 to 5.

Examples of the alkyl group represented by R¹ to R⁷ include a methyl group, an ethyl group, an n-propyl group, an isopropyl group, an n-butyl group, a t-butyl group, an isobutyl group, an s-butyl group, an n-pentyl group, an n-hexyl group, an n-heptyl group, an n-octyl group, an n-nonyl group, and an n-decyl group.

The alkyl group represented by R¹ to R⁷ may have a hydrogen atom substituted with a hydrocarbon group including: a cycloalkyl group such as a cyclopentyl group and a cyclohexyl group; and an aryl group such as a phenyl group.

The number of carbon atoms of a cycloalkyl group represented by R¹ to R⁷ is 3 to 10, preferably 3 to 6.

Examples of the cycloalkyl group represented by R¹ to R⁷ include a cyclopropyl group, a cyclobutyl group, a cyclopentyl group, a cyclohexyl group, and a cycloheptyl group.

The cycloalkyl group represented by R¹ to R⁷ may have a hydrogen atom substituted with a hydrocarbon group including: an alkyl group such as a methyl group, an ethyl group, an n-propyl group, an isopropyl group, an n-butyl group, a t-butyl group, and an isobutyl group; and an aryl group such as a phenyl group.

The number of carbon atoms of an aryl group represented by R¹ to R⁷ is 6 to 10, preferably 6 to 8.

Examples of the aryl group represented by R¹ to R⁷ include a phenyl group, a tolyl group, and a naphthyl group.

The number of carbon atoms of an alkyl group represented by R⁸ is 1 to 5, preferably 1 to 3.

Examples of the alkyl group represented by R⁸ include the methyl group, the ethyl group, the n-propyl group, the isopropyl group, the n-butyl group, the t-butyl group, the isobutyl group, the s-butyl group, and the n-pentyl group.

The number of carbon atoms of a cycloalkyl group represented by R⁸ is 3 to 5, preferably 4 to 5.

Examples of the cycloalkyl group represented by R⁸ include a cyclopropyl group, a cyclobutyl group, and a cyclopentyl group.

The cycloalkyl group represented by R⁸ may have a hydrogen atom substituted with a hydrocarbon group such as the methyl group.

As described above, the group represented by R¹ to R⁸ is a hydrocarbon group and does not include an atom such as an oxygen atom or a halogen atom.

The number of carbon atoms of the divalent group represented by A is 2 to 10, preferably 2 to 5.

Examples of the divalent group represented by A include an ethylene group, a propylene group, a trimethylene group, and a phenylene group.

Examples of the amine compound expressed by the chemical formula (I) may include: ammonia; primary amines such as methylamine, ethylamine, ethylene imine, n-propylamine, isopropylamine, cyclopropylamine, azetidine, 1-methyl aziridine, n-butylamine, t-butylamine, n-pentylamine, n-hexylamine, 2-ethylhexylamine, n-nonyl, n-decylamine, benzylamine, cyclohexylamine, and aniline; secondary amines such as dimethylamine, diethylamine, ethylmethylamine, di-n-propylamine, diisopropylamine, di-n-butylamine, di-t-butyl amine, and di-n-pentylamine; tertiary amines such as trimethylamine, dimethylethylamine, triethylamine, tri-n-propylamine, and triisopropylamine; and cyclic amines such as azacyclobutane, pyrrolidine, piperidine, and hexamethyleneimine.

Examples of the amine compound expressed by the chemical formula (II) may include: ethylenediamine, propylenediamine, tetramethylenediamine, and N,N,N', N'-tetramethylethylenediamine.

Examples of the amine compound expressed by the chemical formula (III) may include: pyridine, 2-methylpyridine, 3-methylpyridine, and 4-methylpyridine.

The amine compounds described above may be used alone or in combination of two or more thereof.

Among the amine compounds described above, the amine compounds with 3 to 5 carbon atoms are preferable, and the amine compounds with 3 or 4 carbon atoms are more preferable. The amine compounds with 3 to 5 carbon atoms may be readily handled. The amine compounds with 5 or less carbon atoms may be easily absorbed to Lewis acids and the like and is highly effective in inhibiting decomposition of fluorinated saturated hydrocarbon.

Preferably, the amine compound described above is chemically adsorbed to the surface of the portion made of metal or metal compound. The chemical absorption of the amine compound suppresses desorption of the amine compound from the surface of the portion made of metal or metal compound. Accordingly, the amine compound mixing into the filtrate obtained by filtering the filtering object using the filter may be reduced, and the effect of inhibiting the decomposition of fluorinated saturated hydrocarbon may be demonstrated for a long time.

Further, the amine compound adsorbed to the surface of the portion made of metal or metal compound has a desorption amount of, in a nitrogen gas atmosphere under the pressure (a gauge pressure) of 0.1 MPa in the temperature of 20°C, preferably 10 ppm by volume or less, more preferably 5 ppm by volume or less. When the desorption amount of the amine compound is equal to or less than the above upper limits, the amine compound mixing into the filtrate obtained by filtering the filtering object may be reduced, and the effect of inhibiting the decomposition of fluorinated saturated hydrocarbon may be demonstrated for a long time.

### Method for Producing Filter

The method for producing the filter according to the present disclosure is a method for producing a filter having a portion that comes into contact with a filtering object and is at least partially made of metal or metal compound. The method for producing the filter according to the present disclosure includes a step (A) of bringing amine compound into contact with a surface of an area that comes into contact with the filtering object and is made of metal or metal compound and, optionally, a step (B) of removing the amine compound physically absorbed to the surface of the area made of metal or metal compound after the step (A).

In the method for producing the filter according to the present disclosure, the amine compound is brought into contact with the surface of the area made of metal or metal compound in step (A). This method yields a filter capable of suppressing decomposition of fluorinated saturated hydrocarbon when filtering a composition with high concentration, i.e., especially at least 99% by volume, preferably at least 99.50% by volume, more preferably at least 99.80% by volume, particularly preferably at least 99.90% by volume of fluorinated saturated hydrocarbon.

It is believed that the filter can inhibit the decomposition of the fluorinated saturated hydrocarbon by virtue of a mechanism similar to that of the filter according to the present disclosure described above.

### [Filter]

Here, the filter having a portion that comes into contact with the filtering object and is at least partially made of metal or metal compound may have any appropriate configuration and may include, for example, a filter body having a filtering object path for flowing a filtering object and a filter element that is accommodated in the filter body and traps fine particles and the like contained in the filtering object flowing in the filtering object path. Each of the filter element and the filter body includes a portion that comes into contact with the filtering object and is at least partially made of metal or metal compound. In particular, examples of the filter having a portion that comes into contact with the filtering object and is at least partially made of metal or metal compound may include: a filter having a filter element made of resin provided within the filtering object path of the filter body made of metal or metal compound; a filter having a filter element made of metal or metal compound provided within the filtering object path of the filter body made of metal or metal compound; and a filter having a filter element made of metal or metal compound provided within the filtering object path of the filter body made of resin.

The metal mentioned above may be any metal including stainless steel or nickel.

The metal compound mentioned above may be any metal compound including aluminum oxide (alumina).

Further, the resin mentioned above may be any resin including polyethylene terephthalate (PTFE).

The filter element described above may be fixedly or detachably provided within the filtering object path of the filter body.

The filter element may have any mesh size capable of demonstrating a desired filtering performance. From the viewpoint of satisfactory removal of impurities such as particles contained in the filtering object, the mesh size of the filter element is preferably at least 1,250 mesh, more preferably at least 4,500 mesh.

### [Step A]

Here, the amine compound that is brought into contact with the surface of the area made of metal or metal compound in step (A) may be any appropriate amine compound including amine compound similar to the amine compound of the filter of the present disclosure described above. In particular, the amine compound may be amine compound with 10 or less carbon atoms (as a total number of carbon atoms) represented by any one of the chemical formulas (I) to (III) set forth above. Among them, amine compounds with 3 to 5 carbon atoms are preferable, and amine compounds with 3 or 4 carbon atoms are more preferable. The amine compounds with 3 to 5 carbon atoms are readily handled. The amine compounds with 5 or less carbon atoms are readily absorbed to Lewis acid and the like and highly effective in inhibiting decomposition of the fluorinated saturated hydrocarbon.

The amine compound may be brought into contact with the area made of metal or metal compound in step (A) by employing any appropriate method, including a method that uses a transfer apparatus such as a pump for continuously or intermittently flowing the amine compound within the filtering object path.

Note that the amine compound may be in a liquid state or in a gaseous state when brought into contact with the surface. The contact of the amine compound may be carried out in a batch operation. Although a mixture of the amine compound and another compound in a small amount (e.g., 10% by mass or less) may be brought into contact with the surface, the amine compound alone is preferably brought into contact.

A pressure (a gauge pressure) for bringing the amine compound into contact may be, for example, -0.100 MPa to -0.001 MPa.

A duration to bring the amine compound into contact may be, for example, 0.1 hour to 10 hours.

A temperature of the amine compound when brought into contact may be, for example, 20 to 100°C.

### [Step B]

In step (B), among the amine compounds adsorbed to the surface of the area made of metal or metal compound in step (A), the amine compound physically absorbed is removed. In step (B), preferably, only the amine compound chemically adsorbed to the surface of the area made of metal or metal compound is left remained. When the amine compound physically adsorbed is removed and the amine compound chemically absorbed is left remained, only the amine compound that is difficult to desorb stays on the surface of the area made of metal or metal compound. Thus, the amine compound mixed into the filtrate obtained by filtering the filtering object using the filter thus prepared may be reduced, and the effect of inhibiting decomposition of fluorinated saturated hydrocarbon may be demonstrated for a long time.

Here, for the removal of the amine compound physically absorbed to the surface of the area made of metal or metal compound, any appropriate method including known absorbate removal methods may be employed. From the viewpoint of efficient removal of the amine compound physically absorbed to the surface while preventing desorption of the amine compound chemically absorbed to the surface of the area made of metal or metal compound, it is preferable in step (B) to remove the amine compound by performing an operation for exposing the surface of the area made of metal or metal compound to a reduced pressure atmosphere. More preferably, the amine compound is removed by alternately repeating the operation for exposing the surface of the portion made of metal or metal compound to a reduced pressure atmosphere and an operation for bringing an inert gas into contact with the surface of the area made of metal or metal compound. Particularly preferably, the amine compound is removed by alternately repeating the operation for exposing the surface of the area made of metal or metal compound to a reduced pressure atmosphere and an operation for bringing a pressurized inert gas into contact with the surface of the area made of metal or metal compound. Here, the number of cycles to alternately repeat the operation for exposing the surface of the area to a reduced pressure atmosphere and the operation for bringing the inert gas into contact with the surface may be, for example, 1 to 100.

The operation for exposing the surface of the area made of metal or metal compound to a reduced pressure atmosphere may be performed by, for example, reducing the pressure within the filtering object path of the filter. The pressure may be reduced to, for example, -0.1 MPa to -0.001 MPa in terms of the gauge pressure. A duration for exposing to the reduced pressure atmosphere may be, for example, 0.01 hour to 10 hours.

The operation for bringing the inert gas into contact with the surface of the area made of metal or metal compound may be performed by, for example, filling the filtering object path of the filter with an inert gas and then exhausting the inert gas, or by continuously flowing an inert gas into the filtering object path. Here, any inert gas including, for example, nitrogen, argon, and helium may be used. A duration for bringing the inert gas into contact with the surface may be, for example, 0.01 hour to 10 hours.

From the viewpoint of facilitating desorption of the amine compound physically absorbed to the surface of the area made of metal or metal compound, the inert gas is preferably in a pressurized state when brought into contact with the surface. The pressure of the pressurized inert gas may be, for example, 0.001 MPa to 0.1 MPa in terms of the gauge pressure.

In step (B), the removal of the amine compound is carried out until the desorption amount of the amine compound in the nitrogen gas atmosphere at the temperature of 20°C under a pressure of 0.1 MPa (in terms of the gauge pressure) reaches preferably 10 ppm by volume or less, more preferably 5 ppm by volume or less. When the desorption amount of the amine compound is equal to or less than the upper limits described above, the amine compound mixing into the filtrate obtained by filtering the filtering object using the filter may be reduced, and the effect of inhibiting the decomposition of fluorinated saturated hydrocarbon may be demonstrated for a long time.

From the viewpoint of reducing the desorption of the amine compound chemically absorbed to the surface, the operation in step (B) described above is normally carried out at a temperature of 100°C or less.

### Dry Etching Apparatus

The dry etching apparatus according to the present disclosure is a dry etching apparatus which uses the dry etching gas obtained by filtering the dry etching gas as the filtering object using the filter of the present disclosure or the filter produced by the method for producing the filter according to the present disclosure. The dry etching apparatus according to the present disclosure may be used for, for example, selective dry etching of a silicon nitride film.

The dry etching apparatus according to the present disclosure includes a material tank for storing a raw material of the dry etching gas (a dry etching composition), a chamber for performing dry etching of the silicon nitride film and the like, and a dry etching gas pipe coupling between the material tank and the chamber via the filter. Optionally, the dry etching apparatus may include a pump for transferring the dry etching gas to the chamber from the material tank, and a vaporizing vessel for vaporizing the raw material (the dry etching composition).

Here, the dry etching gas as the filtering object is generally a composition with high concentration, i.e., at least 99% by volume, preferably at least 99.50% by volume, more preferably at least 99.80% by volume, particularly preferably at least 99.90% by volume of fluorinated saturated hydrocarbon.

The fluorinated saturated hydrocarbon is preferably composed of 3 to 5 carbon atoms.

Fluorinated saturated hydrocarbons whose decomposition is highly effectively suppressed by the amine compound are expressed by C₃H₇F, C₃H₆F₂, C₄H₉F, C₄H₈F₂, C₅H₁₁F, and C₅H₁₀F₂.

Here, the fluorinated saturated hydrocarbons expressed by the molecular formula C₃H₇F include 1-fluoropropane and 2-fluoropropane. The fluorinated saturated hydrocarbons expressed by the molecular formula C₃H₆F₂ include 1,1-difluoropropane, 1,2-difluoropropane, and 2,2-difluoropropane.

The fluorinated saturated hydrocarbons expressed by the molecular formula C₄H₉F include 1-fluorobutane, 2-fluorobutane, 1-fluoro-2-methylpropane, and 2-fluoro-2-methylpropane. The fluorinated saturated hydrocarbons expressed by the molecular formula C₄H₈F₂ include 1,4-difluorobutane, 2,2-difluorobutane, and 2,3-difluorobutane.

Further, the fluorinated saturated hydrocarbons expressed by the molecular formula C₅H₁₁F include 1-fluoropentane, 2-fluoropentane, 3-fluoropentane, 1-fluoro-2-methylbutane, 1-fluoro-3-methylbutane, 2-fluoro-2-methylbutane, 2-fluoro-3-methylbutane, and 1-fluoro-2,2-dimethylpropane. The fluorinated saturated hydrocarbons expressed by the molecular formula C₅H₁₀F₂ include 1,5-difluoropentane, 2,2-difluoropentane, 3,3-difluoropentane, 2,3-difluoropentane, and 2,4-difluoropentane.

Among the fluorinated saturated hydrocarbons mentioned above, fluorinated saturated hydrocarbons whose decomposition is particularly highly effectively suppressed by the amine compound is fluorinated saturated hydrocarbons in which a fluorine atom is not bonded to the carbon atom at the end of the molecule. In particular, examples of the fluorinated saturated hydrocarbons whose decomposition is highly effectively suppressed by the amine compound include 2-fluoropropane, 2,2-difluoropropane, 2-fluorobutane, 2-fluoro-2-methylpropane, 2,2-difluorobutane, 2,3-difluorobutane, 2-fluoropentane, 3-fluoropentane, 2-fluoro-2-methylbutane, 2-fluoro-3-methylbutane, 2,2-difluoropentane, 3,3-difluoropentane, 2,3-difluoropentane, and 2,4-difluoropentane.

The dry etching apparatus according to the present disclosure filters the dry etching gas using the filter according to the present disclosure or the filter produced by the method for producing the filter according to the present disclosure. Thus, the decomposition of the fluorinated saturated hydrocarbon is suppressed. Accordingly, a decrease in the purity of the dry etching gas may be suppressed, enabling excellent dry etching performance.

### Dry Etching Method

The dry etching method according to the present disclosure is a dry etching method that uses the filter according to the present disclosure or the filter produced by the method for producing a filter according to the present disclosure, and may be used for, for example, selective dry etching of the silicon nitride film.

Note that the dry etching gas as the filtering object may be a composition with high concentration of fluorinated saturated hydrocarbon described above, in a manner similar to the dry etching apparatus described above.

The dry etching method according to the present disclosure filters the dry etching gas using the filter according to the present disclosure or the filter produced by the method for producing a filter according to the present disclosure. Thus, the decomposition of the fluorinated saturated hydrocarbon is suppressed. Accordingly, a decrease in the purity of the dry etching gas may be suppressed, enabling excellent dry etching performance.

### EXAMPLES

Hereinafter, the present disclosure will be described in detail by using examples. However, the present disclosure is not limited thereto. In the following description, "%" and "parts" representing a quantity are in terms of by weight, unless otherwise specified.

In the examples and comparative examples, the concentrations of the fluorinated saturated hydrocarbon, the amine compound, and other compounds were measured by the method described below.

### [Concentrations of Fluorinated Saturated Hydrocarbon, Amine Compound, and Other Compounds]

The concentrations were measured by using the following apparatus (a gas chromatograph) under the following conditions.
Apparatus: Agilent® (Agilent is a registered trademark in Japan, other countries, or both) 7890A (manufactured by Agilent Technologies Japan, Ltd.)
Column: "Inert Cap® (Inert Cap is a registered trademark in Japan, other countries, or both) 1" manufactured by GL Sciences Inc., with a length of 60 m, an inner diameter of 0.25 mm, and a film thickness of 1.5 µm
Column temperature: maintained at 40°C for 15 minutes, heated to 240°C at a speed of 100 °C./min, and then maintained at 240°C for 10 minutes
Injection temperature: 80°C.
Carrier gas: nitrogen
Split ratio: 40/1
Detector: FID

### Example 1

### [Preparation of Filter]

To a filter (model type PGF-3-02SW·PC07 manufactured by PURELON JAPAN CO., LTD.) including a filter body of SUS316L and a filter element made of alumina ceramic, isopropylamine as the amine compound at a temperature of 20°C was introduced at a flow rate of 200 mL/min while a vacuum pump was used such that a pressure on an outlet side of the filter was -0.09 MPa (the gauge pressure). Next, an operation for setting the temperature within the filtering object path of the filter to 20°C and reducing the pressure of the atmosphere within the filtering object path to -0.1 MPa (the gauge pressure) for 1 minute, and an operation for sealing nitrogen at the temperature of 20°C under the pressure of 0.1 MPa (gauge pressure) in the filtering object path of the filter and then exhausting the nitrogen after 1 minute were alternatingly repeated for 20 cycles. Then, concentration of amine compound contained in the exhausted gas in the 20th cycle was measured. A result is illustrated in Table 1.

### [Evaluation of Filter Performance]

A dry etching gas (a filtering object) containing 2-fluorobutane at concentration of 99.97% by volume was introduced to a filter heated to 50°C at a flow rate of 25 mL/min for 120 minutes. Subsequently, concentration of 2-fluorobutane (fluorinated saturated hydrocarbon) was measured.

Then, whether the concentration of 2-fluorobutane has changed between before and after passing through the filter was determined. A result is illustrated in Table 1.

### Examples 2 and 3

A filter was prepared in a manner similar to the example 1, except for using ethylmethylamine (the example 2) or dimethylethylamine (the example 3) as the amine compound, and then subjected to the performance evaluation. Results are illustrated in Table 1.

### Comparative Example 1

A filter was prepared in a manner similar to the example 1, except for being untreated without using amine compound, and then subjected to the performance evaluation. Results are illustrated in Table 1.

### Comparative Examples 2 and 3

A filter was prepared in a manner similar to the example 1, except for using chloroform (the comparative example 2) or methanol (the comparative example 3) in place of amine compound, and then subjected to the performance evaluation. Results are illustrated in Table 1.

**[Table 1]**

| | | | Example 1 | Example 2 | Example 3 | Comparative Example 1 | Comparative Example 2 | Comparative Example 3 |
|---|---|---|---|---|---|---|---|---|
| Exhausted Gas | Amine Compound | Isopropylamine [ppm by volume] | 10 or less | - | - | - | - | - |
| | | Ethylmethylamine [ppm by volume] | - | 10 or less | - | - | - | - |
| | | Dimethylethylamine [ppm by volume] | - | - | 10 or less | - | - | - |
| | Other Compounds | Chloroform [ppm by volume] | - | - | - | - | 10 or less | - |
| | | Methanol [ppm by volume] | - | - | - | - | - | 10 or less |
| Filtering Object | Fluorinated Saturated Hydrocarbon | 2-Fluorobutane [% by volume] | 99.97 | 99.97 | 99.97 | 99.97 | 99.97 | 99.97 |
| Filtered Gas | Fluorinated Saturated Hydrocarbon | 2-Fluorobutane [% by volume] | 99.97 | 99.97 | 99.97 | 99.62 | 99.64 | 99.70 |

It can be seen from Table 1 that the filters treated with amine compound in the examples 1 to 3 reduced decomposition of 2-fluorobutane more than the untreated filter in the comparative example 1 and the filters treated with other compounds in the comparative examples 2 and 3.

### INDUSTRIAL APPLICABILITY

The present disclosure may provide a filter having a portion that comes into contact with a filtering object to be filtered and is at least partially made of metal or metal compound, wherein the filter is capable of inhibiting the decomposition of fluorinated saturated hydrocarbon when filtering a composition containing high concentration of fluorinated saturated hydrocarbon.

The present disclosure may also provide a dry etching apparatus having a filter that is capable of inhibiting decomposition of fluorinated saturated hydrocarbon, and a dry etching method that uses a filter capable of inhibiting the decomposition of fluorinated saturated hydrocarbon.

## Claims

1. A filter comprising:
a portion made of metal or metal compound for coming into contact with a filtering object; and
amine compound absorbed to a surface of the portion.

2. The filter according to claim 1,
wherein the amine compound is chemically adsorbed to the surface.

3. The filter according to claim 1 or 2,
wherein a desorption amount of the amine compound in a nitrogen gas atmosphere at a temperature of 20°C and a gauge pressure of 0.1 MPa is 10 ppm by volume or less.

4. The filter according to any one of claims 1 to 3,
wherein a mesh size of a filter element is at least 1,250 mesh.

5. The filter according to any one of claims 1 to 4,
wherein the amine compound is composed of amine with 3 to 5 carbon atoms.

6. A method for producing a filter having a portion that comes into contact with a filtering object and at least partially includes an area made of metal or metal compound, the method for producing a filter comprising:
a step (A) of bringing amine compound to come into contact with a surface of the area.

7. The method for producing a filter according to claim 6,
further comprising a step (B) of removing amine compound physically absorbed to the surface after the step (A).

8. The method for producing a filter according to claim 7,
wherein the step (B) includes an operation for exposing the surface to a reduced pressure atmosphere.

9. The method for producing a filter according to claim 7 or 8,
wherein the step (B) includes alternate repeating of the operation for exposing the surface to a reduced pressure atmosphere and an operation for bringing an inert gas into contact with the surface.

10. The method for producing a filter according to any one of claims 6 to 9,
wherein a mesh size of a filter element of the filter is at least 1,250 mesh.

11. The method for producing a filter according to any one of claims 6 to 10,
wherein amine compound is composed of amine with 3 to 5 carbon atoms.

12. A dry etching apparatus for performing dry etching by using a dry etching gas filtered by a filter,
wherein the filter is a filter according to any one of claims 1 to 5.

13. A dry etching method for performing dry etching by using a dry etching gas filtered by a filter according to any one of claims 1 to 5.
